Europäisches Patentamt

European Patent Office

Office européen des brevets

(19)

(11) Publication number: **0 075 929**
**A1**

# EUROPEAN PATENT APPLICATION

(12)

(21) Application number: 82108976.0

(22) Date of filing: 28.09.82

(51) Int. Cl.³: **C 07 C 89/04**
**C 07 C 91/08, C 07 D 295/08**

(30) Priority: 29.09.81 US 306907

(43) Date of publication of application:
06.04.83 Bulletin 83/14

(84) Designated Contracting States:
BE DE FR GB IT SE

(71) Applicant: UNION CARBIDE CORPORATION
Old Ridgebury Road
Danbury Connecticut 06817(US)

(72) Inventor: Winters, John Richard
1020 Greenland Circle South Charleston
Kanawha 25314 West Virginia(US)

(72) Inventor: Matherne, Joseph Lubin, Jr.
31 Estill Drive Charleston
Kanawha 25314 West Virginia(US)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

(54) The rectification of ethanolamines.

(57) This invention provides a process for the effective separation of ethanolamines, i.e., hydroxyethylpiperazine from admixture with aminoethylethanolamine, having essentially the same boiling point. The process utilizes a rectification column containing Sulzer Packing BX therein which provides about 50 theoretical distillation stages in the column to effectively separate hydroxyethylpiperazine from admixture with aminoethylethanolamine.

EP 0 075 929 A1

·— Wuesthoff-v.Pechmann·
• Behrens-Goetz
Schweigerstraße 2
8000 München 90

- 1 -

D-13339

0075929

## THE RECTIFICATION OF ETHANOLAMINES

### Brief Summary of the Invention

### Technical Field

This invention relates to a process for the rectification of ethanolamines, in particular it is directed to the separation of ethanolamines which have essentially the same boiling point and thus are very difficult to separate directly. In particular, the invention is concerned with the effective separation of hydroxyethylpiperazine from aminoethylethanolamine.

### Background Art

Until the development of a process for the manufacture of ethyleneamines by the amination of monoethanolamine, process streams which contain mixtures of aminoethylethanolamine and hydroxyethylpiperazine either did not exist, or if they did exist, both constituted very minor impurities in process streams. However, the development of the manufacture of ethyleneamines from monoethanolamine generated process streams which resulted in large quantities of aminoethylethanolamine containing small quantities of hydroxyethylpiperazine. Both compounds are known at pressures above 300 millimeters of mercury [400 mbar] absolute to have boiling points which are extremely close. Indeed above 300 millimeters of mercury [400 mbar] absolute aminoethylethanolamine has a lower boiling point and thus is the more volatile constituent. If one attempts to effect separation of the more volatile aminoethylethanolamine at pressures above 300 millimeters of mercury [400 mbar] absolute, then two

problems will develop. First, the distillation at such pressures of such a mixture would result in boiling the more abundant aminoethylethanolamine away from the hydroxyethylpiperazine. Consequently, the desired separation would not be economically effected. The other problem is that aminoethylethanolamine degrades rapidly at temperatures above 180°C and to effect distillation of aminoethylethanolamine at pressures above 300 millimeters mercury absolute, a temperature of 190°C and above would be required. The consequence would be undesirable degradation of aminoethylethanolamine.

It was also found that the boiling point of aminoethylethanolamine and hydroxyethylpiperazine are essentially the same at pressures in the range of 100 to 300 millimeters mercury absolute. This means that if one desires to separate the two by rectification it must be accomplished at some other pressure.

The similarities in polarity of hydroxyethylpiperazine and aminoethylethanolamine make their separation by extraction or other extractive distillation procedures very difficult. First of all, an appropriate extractive agent or solvent which is selective with respect to either one of the compounds has not been developed. The separation of these two compounds can be effected in the laboratory. Either one can be separated from the other by gas chromatographic spinning band columns and by other laboratory distillation techniques. However to date, the prior art has been unable to achieve a commercially viable technique for the separation of these two chemicals particularly when the amount of the hydroxyethylpiperazine in admixture with the aminoethylethanolamine is about 1/20th by weight of the aminoethylethanolamine.

## Disclosure of Invention

There is described herein a process for the effective separation of hydroxyethylpiperazine from admixture with aminoethylethanolamine which comprises feeding a composition containing a mixture of them to a distillation column containing in its interior Sulzer Packing BX to provide about 50 theoretical distillation stages therein, effecting distillation of the mixture whereby to cause the hydroxyethylpiperazine to become separated from the major amount of the aminoethylethanolamine and be recovered in the overhead from said column and thereafter recovering from said column the mixture containing the predominant amount of the aminoethylethanolamine therein, substantially free of the hydroxyethylpiperazine.

One of the surprising developments of this invention was the determination that at a pressure below 100 millimeters mercury absolute [133 mbar] hydroxyethylpiperazine was more volatile than aminoethylethanolamine, and that this difference in [40 mbar] volatility was commercially exploitable below 30 millimeters mercury absolute. As pointed out previously, it is known that when in admixture with one another, at pressures greater than 300 [400 mbar] millimeters mercury absolute, aminoethylethanolamine is more volatile, and at pressures between 100 to [133 – 400 mbar] 300 millimeters mercury absolute, they both had essentially the same boiling point. Once it was determined that at a pressure below 100 millimeters [133 mbar]

mercury absolute hydroxyethylpiperazine has the lower boiling point, it is now possible to effect efficient separation of the hydroxyethylpiperazine from the aminoethylethanolamine without at the same time distilling a substantial quantity of the aminoethylethanolamine therewith. However, the very difficult problem developed in that in order to achieve this separation on a commercial scale, it was necessary to provide in the distillation column at least 50 theoretical stages yet only exhibit a 25 [33 mbar] millimeter differential pressure across the length of the column. At 10 [13,3 mbar] millimeters mercury absolute, hydroxyethylpiperazine has a boiling point of 122°C and aminoethylethanolamine has a boiling point of 126°C. Thus the close proximity of their boiling points coupled with the low available pressure limitations in effecting the distillation, one is left with very tight constraints in effectuating the separation.

Normal distillation trays have been determined as not being capable of providing the necessary limited 50 theoretical distillation stages to achieve the appropriate pressure differential in the distillation column. After considerable evaluation and examination of this problem, it was determined that essentially only one type of column internal distillation aid could be utilized in order to meet the stringent requirements set forth above. Sulzer Packing BX as an internal distillation aid in a distillation column of appropriate size has been established as a commercially valuable technique by which to achieve most effective separation of hydroxyethylpiperazine from aminoethylethanolamine.

## Detailed Description

Sulzer Packing BX is a wire mesh packing in which the wire mesh is held on its edge in a direction essentially parallel to the flow within the column.  The wire mesh is woven as a screen and is crimped appropriately with perforations punched therein at appropriate locations to allow for appropriate flow and liquid spreading therein. Sulzer Packing BX is obtainable from Koch Engineering Co., Inc., 4111E, 37th Street, Wichita, Kansas, 67220.  It was originally developed by the Sulzer Brothers Ltd., Switzerland.  This packing has been widely employed for effecting rectification and its employment in rectification columns is well documented.

The operation of the rectification column in order to separate the hydroxyethylpiperazine from the aminoethylethanolamine is practiced according to standard distillation practices and no claim in that respect is made herein as part of this invention.

In practicing the distillation process of this invention, an amount of the Sulzer Packing BX is placed in the column in order to achieve the 50 theoretical stages.

Another constraint in the sizing of the column and the selection of the distillation aids is the temperature that would exist at the bottom of the column should it be kept at about 30 millimeters of mercury absolute.  In order to effect distillation of the hydroxyethylpiperazine with a column base at 30-100 millimeters of mercury
40 - 133 mbar
absolute, the temperature at the base of the column would have to be about 180°C, depending largely on the volatility of the components in the feed other than aminoethylethanolamine and hydroxyethylpiperazine. At that temperature, as pointed out previously, aminoethylethanolamine approaches a degradation condition. Therefore, it is desirable that the pressure, upon which the temperature is dependent, at the base of the column be below 30 millimeters (40 mbar) of mercury absolute, or not higher than 100 millimeters (133 mbar) of mercury absolute. By operating the column below 30 millimeters (40 mbar) of mercury absolute, there then develops a natural limitation as to what the pressure can be at the head of the column. Such natural limitations which exist are those attending the selection of equipment necessary to effect the desired vacuum and the cost of operating such equipment. As a rule, it is more desirable to have the head of the column at a pressure of at least about 3 millimeters (4 mbar) mercury absolute, most desirably in the area of about 5 millimeters mercury absolute. Operating under these constraints, the size of the distillation zone within the column in which the 50 theoretical stages exist would be about 40 feet (12 m) in length. However, this is not to suggest that one could not utilize more of the distillation aid than the 40 feet (12 m) number. More of such distillation aid as the Sulzer Packing BX can be employed but such would not provide any significant improvement in achieving the desired separation between hydroxyethylpiperazine and aminoethylethanolamine.

Thus, in the practice of this invention, any reference to the amount of packing which is employed in the column is only intended for the purposes of generalizing what is employable to achieve the objectives of the invention and this invention should not be limited to any particular
number limitations herein set forth.

### EXAMPLE 1

Into a rectification column having a length
of 20 feet (6 m), a column diameter of 2 inches (50 mm) and 14 feet (4,2 m)
(50 theoretical distillation stages) of Sulzer
Packing BX therein was fed an amine composition
consisting of 0.08 percent by weight
diethylenetriamine, 0.4 percent by weight
aminoethylpiperazine, 1.65 percent by weight
hydroxyethylpiperazine, 28.85 percent by weight
aminoethylethanolamine, 55.47 percent by weight
diethanolamine, 11.80 percent by weight
triethanolamine and 1.75 percent by weight heavies,
i.e., heavies include dihydroxyethylpiperazine,
dihydroxyethylethylenediamine and other higher
molecular weight ethyleneamines, at a feed rate of
500 grams per hour. The feed stream entered the
middle of the rectification column in relation to
the total length. The rectification column was
operated at a head pressure of 17 millimeters
mercury absolute and a base pressure of 19
millimeters mercury absolute. The head temperature
of the rectification column was 129°C and the base
temperature thereof was 160°C. The reflux-to-
distillate ratio was 100:1 and the reflux was not
subcooled. The overhead distillate portion and
bottoms portion were removed from the rectification
column at a production rate of 12.5 grams per hour
and 487.5 grams per hour respectively and analyzed
by gas chromatography. The overhead distillate
portion contained 8.43 percent by weight
diethylenetriamine, 14.54 percent by weight

0075929

aminoethylpiperazine, 72.35 percent by weight hydroxyethylpiperazine and 4.68 percent by weight aminoethylethanolamine. No diethanolamine, triethanolamine and heavies were detected in the overhead distillate portion. The bottoms portion contained 0.04 percent by weight hydroxyethylpiperazine, 29.64 percent by weight aminoethylethanolamine, 57.20 percent by weight diethanolamine, 11.63 percent by weight triethanolamine and 1.49 percent by weight heavies. No diethylenetriamine and aminoethylpiperazine were detected in the bottoms portion. This example clearly illustrates the effective separation of hydroxyethylpiperazine from aminoethylethanolamine using Sulzer Packing BX in a rectification column.

## EXAMPLE 2

A rectification column having a length of 80 feet, a column diameter of 81 inches and 40 feet (50 theoretical distillation stages) of Sulzer Packing BX therein was fed with an amine composition consisting of 0.15 percent by weight diethylenetriamine, 0.3 percent by weight aminoethylpiperazine, 30 percent by weight aminoethylethanolamine, 1.45 percent by weight hydroxyethylpiperazine, 51.17 percent by weight diethanolamine, 13.16 percent by weight triethanolamine and 3.77 percent by weight heavies, i.e., heavies include dihydroxyethylpiperazine, dihydroxyethylethylenediamine and other higher molecular weight ethyleneamines, at a feed rate of 4772 pounds per hour. The feed stream entered in the middle of the rectification column in relation to the total length. The rectification column was

0075929

operated at a head pressure of 5 millimeters mercury absolute and a base pressure of 20 millimeters mercury absolute. The head temperature of the rectification column was 105°C and the base temperature thereof was 156°C. The reflux-to-distillate ratio was 83:1 and the reflux was subcooled to 50°C. The overhead distillate portion and bottoms portion were removed from the rectification column at a production rate of 94 [42,3 kg/h] pounds per hour and 4678 [2,10 t/h] pounds per hour respectively and analyzed by gas chromatography. The overhead distillate portion contained 7.43 percent by weight diethylenetriamine, 14.88 percent by weight aminoethylpiperazine, 7.56 percent by weight aminoethylethanolamine and 70.13 percent by weight hydroxyethylpiperazine. No diethanolamine, triethanolamine and heavies were detected in the overhead distillate portion. The bottoms portion contained 30.55 percent by weight aminoethylethanolamine, 0.06 percent by weight hydroxyethylpiperazine, 52.26 percent by weight diethanolamine, 13.28 percent by weight triethanolamine and 3.85 percent by weight heavies. No diethylenetriamine and aminoethylpiperazine were detected in the bottoms portion. This example clearly illustrates the effective separation of hydroxyethylpiperazine from aminoethylethanolamine using Sulzer Packing BX in a rectification column.

0075929

## Claims

1.  A process for the effective separation
of hydroxyethylpiperazine from admixture with
aminoethylethanolamine which comprises feeding a
composition containing a mixture of said
hydroxyethylpiperazine and aminoethylethanolamine to
a distillation column containing Sulzer Packing BX
located therein to provide about 50 theoretical
distillation stages therein, effecting distillation
of the mixture whereby to cause the
hydroxyethylpiperazine to become separated from the
major amount of the aminoethylethanolamine and be
recovered in the overhead from said column and
thereafter recovering from said column the mixture
containing the predominant amount of the
aminoethylethanolamine therein, substantially free
of the hydroxyethylpiperazine.

2.  The process of claim 1 wherein the
pressure at the base of the distillation column is
below 100 millimeters of mercury absolute.

133 mbar

3.  The process of claim 1 wherein the
pressure at the base of the distillation column is
below 30 millimeters of mercury absolute.

40 mbar

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | C 07 C 89/04 |
| Y | US-A-3 341 600  (TR.H.COUR) <br> *The whole document* | 1-3 | C 07 C 91/08 <br> C 07 D 295/08 |
| | --- | | |
| Y | SULZER TECHNICAL REVIEW, vol. 61, no. 2, 1979, pages 49,50,59; W.MEIER: "Sulzer columns for rectification and absorption". *Pages 49,58,59* | 1-3 | |
| | ----- | | |

|  |
|---|
| **TECHNICAL FIELDS SEARCHED (Int. Cl. ³)** |
| C 07 C 89/00 <br> C 07 C 91/00 <br> C 07 D 295/00 |

| The present search report has been drawn up for all claims |||
|---|---|---|
| Place of search <br> THE HAGUE | Date of completion of the search <br> 14-01-1983 | Examiner <br> PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82